# FASCICULE DE BREVET EUROPEEN

(11) **EP 1 201 206 B1**
(45) Date de publication et mention de la délivrance du brevet: **20.07.2005**
(21) Numéro de dépôt: 01402643.9
(22) Date de dépôt: 12.10.2001
(51) Int. Cl.: A61F 2/38

(54) **Composant fémoral incliné**
Geneigte Femurkomponente
Inclined femoral component

(30) Priorité: 18.10.2000 FR 0013313
(43) Date de publication de la demande: 02.05.2002
(73) Titulaire: Aesculap Société par actions simplifiée, 52000 Chaumont (FR)
(72) Inventeur: Biegun, Jean-François, 52000 Chaumont (FR); Marceaux, Pascal, 52000 Chaumont (FR)
(74) Mandataire: Eidelsberg, Olivier

(56) Documents cités:
- EP-A- 0 600 806
- WO-A-95/14444
- DE-U- 8 911 095
- US-A- 5 326 361
- US-A- 5 681 354
- US-A- 5 824 105
- US-A- 6 013 103

## Description

La présente invention concerne un composant fémoral, destiné à coopérer avec un plateau tibial, éventuellement avec interposition d'un insert ou ménisque tibial posé sur le plateau tibial, ainsi qu'une prothèse du genou comportant un composant fémoral de ce genre.

Les composants fémoraux connus de l'art antérieur comportent une trochlée et au moins un, de préférence deux, condyles, un trajet trochléen étant défini par la trochlée dans la surface extérieure du composant, et un ensemble de faces planes intérieures destinées à venir en contact avec des faces résectées correspondantes de l'extrémité distale d'un fémur étant définies dans la surface intérieure du composant, lesdites faces planes intérieures définissant entre elles des arêtes intérieures.

Lorsque l'on pose des prothèses tibiales du genre mentionné précédemment, et notamment lorsque l'on souhaite ancrer un composant fémoral du genre mentionné précédemment à l'extrémité distale d'un fémur, on va d'abord effectuer ce que l'on appelle une coupe ou résection de l'extrémité du fémur. En général, cette résection est effectuée de sorte que l'extrémité du fémur comporte plusieurs faces séparées entre elles par des arêtes. Une des faces est sensiblement perpendiculaire à l'axe du fémur, tandis que les autres faces sont inclinées par rapport à ladite une face.

De même, pour la pose de la prothèse, le tibia va également être réséqué suivant une face à l'extrémité proximale du tibia qui est sensiblement perpendiculaire à l'axe longitudinal du tibia.

On définit, pour une prothèse de genou donné, le côté médial et le côté latéral de cette prothèse. Le plan médial latéral est le plan parallèle à l'axe du tibia et comportant également l'axe de l'autre tibia. Par rapport à une prothèse donnée, le côté médial est alors le côté de la prothèse se trouvant du côté de l'autre prothèse ou de l'autre genou sain du patient et le côté latéral est le côté se trouvant à l'extérieur du patient.

Lorsque l'on découpe le tibia suivant ce que l'on appelle une coupe neutre comme défini précédemment, c'est-à-dire en formant une face plane perpendiculaire à l'axe du tibia, on va retirer plus d'os de la partie supérieure latérale du tibia que de la partie supérieure médiale du tibia. En revanche, la découpe neutre du fémur va retirer la même quantité d'os, des deux condyles médial et latéral. Par conséquent, avant de poser et de fabriquer la prothèse, on s'aperçoit qu'il existe entre le tibia réséqué et le fémur réséqué non pas un parallélogramme mais un volume trapézoïdal, la distance du tibia au fémur perpendiculairement à la face réséquée tibiale du côté latéral étant supérieure à la même distance du côté médial.

L'existence de cet espace trapézoïdal n'est pas souhaitable. En effet, cela peut se traduire par un ligament collatéral médial trop serré ou un ligament collatéral latéral trop lâche. Par conséquent, pour ne pas avoir cet espace trapézoïdal, on a pensé, lors de la pose du fémur, à réaliser une rotation externe du fémur. En tournant le fémur, on retire plus d'os au niveau médial qu'au niveau latéral et on crée un espace rectangulaire. Cependant, effectuer cette rotation externe du fémur et notamment effectuer une coupe telle que plus d'os médial que d'os latéral soit découpé présente les inconvénients suivants :
1) Tout d'abord, lorsque le fémur est tourné externement, le trajet trochléen proximal est décalé latéralement tandis que la rainure est ramenée distalement vers le côté médial. Ainsi, lorsque le genou est fléchi, une fois la prothèse posée, la trochlée traverse la ligne médiane et se déplace médialement. Le fait de "médialiser" le trajet trochléen peut être un facteur contribuant à un mauvais positionnement de la trochlée lors de son mouvement, ce qui peut se traduire par des douleurs, une fracture, un relâchement ou une usure.
2) En outre, lorsque le fémur est tourné externement, il devient raisonnable également de tourner extérieurement le tibia également pour aligner le fémur et le tibia en position étendue du genou. Il peut en résulter alors que la plaque de base de la prothèse du tibia s'étend au-delà de l'os, de sorte que le chirurgien doit prévoir, pour que cela n'arrive pas, un composant tibial plus petit. Si une prothèse du tibia plus petite est utilisée, la couverture générale de l'os sera diminuée, ce qui n'est pas bon non plus.
3) En outre, les composants fémoraux du tibia doivent être alignés en rotation sur tout un domaine de déplacement afin de réduire l'usure du polyéthylène de l'insert se trouvant entre eux. Lorsque les deux composants fémoral et tibial sont tournés extérieurement, la zone de contact entre le fémur et le tibia est rendue maximale en extension, mais est réduite au fur et à mesure que le genou se fléchit, c'est-à-dire la congruence devient moins bonne.
   Si le composant tibial est placé suivant une rotation neutre avec le composant fémoral qui, lui, est tourné extérieurement, la congruence est petite en flexion mais grande en extension. Il en résulte que l'aire de contact est rendue maximale en flexion, mais réduite en extension. Il ressort donc de ce qui précède qu'indépendamment de l'orientation du composant tibial, lorsque le fémur est tourné extérieurement, la congruence ne peut pas être conservée dans tout le domaine de flexion. Cela devient un facteur ou paramètre très important avec des conceptions de genou à conformation élevée.
4) Le fait d'effectuer une rotation externe du fémur retire plus d'os du côté latéral antérieur et augmente la probabilité de la formation d'une encoche. Cela peut prédisposer le fémur à une fracture, ce qui n'est pas souhaitable.
5) Enfin, une extraction non appropriée d'os du fémur antérieur médial peut créer un interstice entre l'implant et l'os et peut compromettre la bonne situation de l'implant.

Parmi les prothèses de l'art antérieur on connaît notamment celle décrite dans le modèle d'utilité allemand N° G 8911095.1 au nom de Miehlke. Dans cette prothèse, les arêtes des cages sont parallèles au plan tangentiel aux condyles dans leur partie la plus distale et le trajet trochléen y est "incliné" par rapport à la perpendiculaire à ce plan tangentiel (ou ligne de référence définie par les condyles lorsqu'ils reposent sur un plan horizontal).

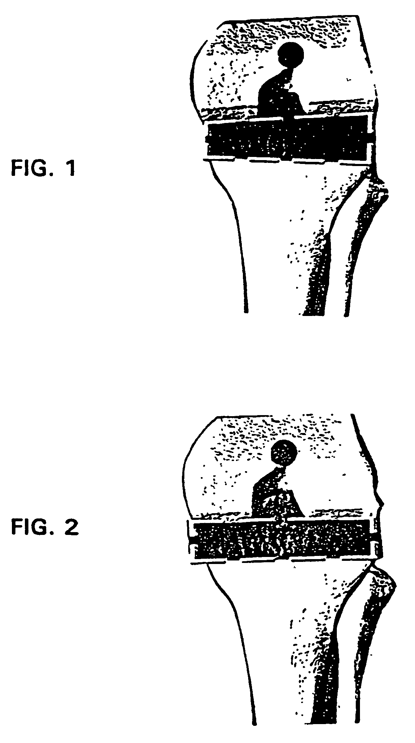

On connaît également celle décrite dans le brevet américain Ries N° 5.824.105. Les arêtes y sont également parallèles au plan tangentiel aux condyles dans leur partie la plus distale et le trajet trochléen est perpendiculaire à ce plan, un insert biseauté étant disposé entre le plateau tibial et les condyles pour permettre de remplir au mieux l'espace trapezoïdal de la figure 1.

Une autre prothèse fémorale de l'art antérieur est celle décrite dans le brevet américain N° 5.326.361 au nom de Hollister. Dans ce document la cage (les arêtes) est inclinée par rapport au plan tangentiel aux condyles (ou ligne de référence) et le trajet de trochlée est également incliné par rapport à la perpendiculaire au plan tangentiel.

Enfin, la prothèse décrite dans US-A-6.013.103 au nom de Kaufman prévoit une cage parallèle au plan tangentiel aux condyles et un trajet de trochlée incliné par rapport à la perpendiculaire au plan tangentiel.

La présente invention vise à pallier les inconvénients mentionnés ci-dessus et notamment propose un composant fémoral nouveau qui permet, sans avoir à effectuer une rotation externe du fémur lors de sa résection, de résoudre néanmoins le problème lié à la résection neutre du tibia, c'est-à-dire la présence d'un interstice trapézoïdal entre le tibia réséqué et le fémur réséqué.

Grâce à l'invention, on n'a pas à supporter les inconvénients liés à la rotation externe du fémur, car celle-ci n'est plus à réaliser, et en même temps, le ligament collatéral à la prothèse n'a pas de tension variable entre le côté médial et le côté latéral de la prothèse.

Suivant l'invention, un composant fémoral d'une prothèse du genou, comportant une partie de trochlée et au moins un, de préférence deux, condyle(s), un trajet trochléen étant défini dans la partie de trochlée dans la surface extérieure du composant, et un ensemble de faces planes intérieures destinées à venir en contact avec des faces réséquées correspondantes de l'extrémité distale d'un fémur définissant une cage ouverte intérieure dans la surface intérieure du composant, lesdites faces planes intérieures définissant entre elles des arêtes intérieures, les condyles définissant une ligne de référence lorsqu'ils sont posés sur un plan horizontal, correspondant à la ligne de contact avec ce plan, est caractérisé en ce que la projection perpendiculaire du trajet trochléen dans le plan médio-latéral est perpendiculaire à la ligne de référence et les arêtes, en projection orthogonale sur le plan médio-latéral, sont inclinées d'un angle d'inclinaison non nul par rapport à la ligne de référence.

En prévoyant ainsi d'"incliner" les faces internes du composant fémoral par rapport au plan tibial sur lequel va le reposer les condyles, c'est-à-dire d'incliner les cages, on peut remplir parfaitement ledit espace trapézoïdal et donc avoir une tension égale de part et d'autre de la prothèse pour les ligaments collatéraux à ladite prothèse, sans avoir pour se faire à effectuer une rotation externe du composant du fémur, rotation externe qui elle-même sinon présenterait des inconvénients importants.

Suivant un mode de réalisation préféré de l'invention, la cage ouverte intérieure comporte cinq faces planes.

Suivant un mode de réalisation préféré de l'invention, les condyles sont extérieurement de forme sphérique.

Suivant un mode de réalisation préféré de l'invention, l'angle d'inclinaison correspond à l'angle dont doit être tourné le fémur dans le cas de la méthode dite de la rotation externe du fémur, de sorte que cette méthode n'est plus nécessaire.

La présente invention vise également un couple de composants fémoraux tels que mentionnés précédemment, à savoir un composant fémoral gauche et un composant fémoral droit, l'angle d'inclinaison est calculé, dans le cas de la prothèse gauche, dans le sens des aiguilles d'une montre, et pour la prothèse droite dans le sens inverse des aiguilles d'une montre.

La présente invention vise également une prothèse totale du genou comportant un composant tel que décrit précédemment coopérant avec un plateau tibial avec éventuellement interposition d'un ménisque ou insert tibial notamment en polyéthylène.

Aux dessins, donnés uniquement à titre d'exemple, il est décrit un mode de réalisation de la présente invention.

La figure 1 est une vue d'un genou ayant subi une résection du fémur et une résection du tibia en position fléchie du genou, et sans rotation externe du fémur.

La figure 2 est une vue identique à celle de la figure 1, mais dans laquelle le fémur a subi une rotation externe.

La figure 3 est une vue de côté d'un composant fémoral suivant l'invention, vue de la droite de la prothèse de la figure 4.

La figure 4 est une vue en plan médio-latéral du composant de la figure 3, dans le cas d'une prothèse gauche, et

La figure 5 est une vue en plan médio-latéral de la prothèse de la figure 3 dans le cas d'une prothèse droite.

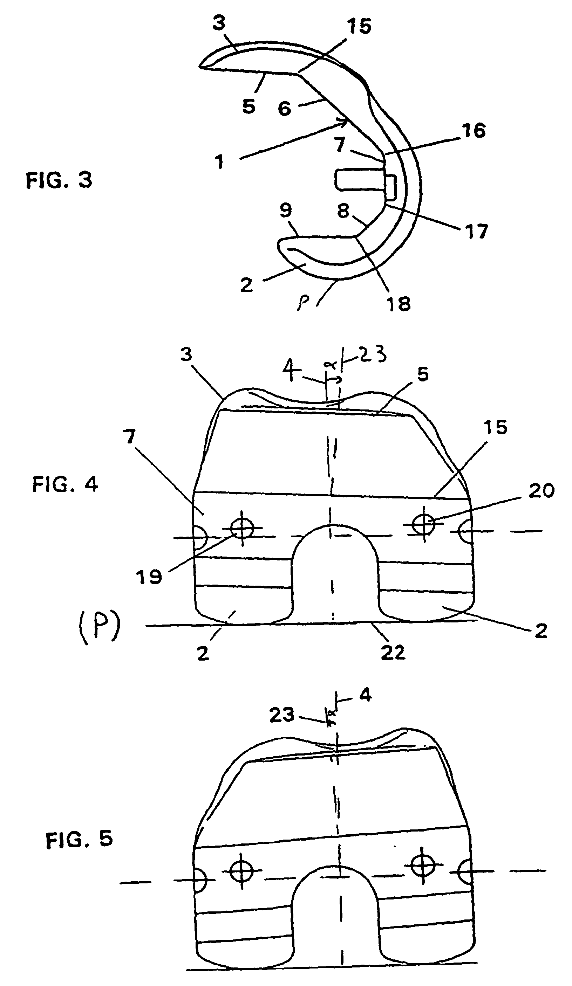

A la figure 3, le composant fémoral 1 comporte deux condyles 2 et une trochlée 3. La trochlée 3 définit un trajet trochléen dont on voit la projection 4 dans le plan de la figure 4, qui est le plan médio-latéral. La surface extérieure des condyles 2 a, dans le plan de la figure 3 qui est aussi le plan antéro-postérieur, une forme circulaire, et il en est de même pour la trochlée 3, le trajet trochléen 4 ayant une forme circulaire. Il est défini à l'intérieur du composant 1 fémoral cinq faces planes 5, 6, 7, 8 et 9, définissant une cavité ouverte. Ces cinq faces planes 5, 6, 7, 8, 9 sont séparées les unes des autres par des arêtes 15, 16, 17, 18. De la face 7 intérieure, sont issus deux plots 19 et 20 sur lesquels est destiné à s'ancrer le fémur réséqué. Les faces intérieures 5, 6, 7, 8, 9 correspondent en dimension et inclinaison aux faces réséquées dans le fémur de la figure 1. Dans le plan médio-latéral, les arêtes, et notamment l'arête 15, est inclinée de α = 3°, par rapport à la perpendiculaire 22 à la projection 4 perpendiculaire du trajet trochléen dans le plan de la figure 4 (plan médio-latéral). Cette inclinaison α est variable en fonction du fémur de chaque personne et peut être comprise en général, entre 1 et 10°, de préférence entre 2° et 5°. Vu de l'extérieur, le composant fémoral suivant l'invention a le même aspect que les prothèses de l'art antérieur. Cependant, la cage intérieure a été formée tournée par rapport aux cages de l'art antérieur. Les cages de l'art antérieur étaient formées "parallèlement" à la prothèse tandis que maintenant, cette cage intérieure est formée en étant légèrement inclinée par rapport à la prothèse.

La figure 4 représente une prothèse gauche, dans le plan parallèle au plan tibial, l'angle entre la projection dans le plan médio-latéral du trajet trochléen et la perpendiculaire 23 dans ce même plan à la projection de l'arête 15 est de moins 3°, la rotation s'effectuant dans le sens trigonométrique négatif.

A la figure 5, cet angle est de plus 3°, la rotation s'effectuant dans le sens inverse des aiguilles d'une montre, c'est-à-dire le sens positif trigonométrique. Il s'agit donc d'une prothèse de jambe droite.

La figure 1 représente les os du fémur et du tibia tels que réséqués avant la pose de la prothèse, le fémur n'ayant pas subi de rotation externe. La figure 2 représente les mêmes os réséqués dans le cas différent de l'application de la présente invention, où le fémur a subi une rotation externe.

Les angles d'inclinaison des deux composants ont la même valeur absolue.

La ligne de contact des condyles avec une surface horizontale sur laquelle ils sont posés est la ligne de référence. La perpendiculaire 22 à la projection 4 perpendiculaire du trajet trochléen dans le plan de la figure 4 est également la ligne de référence 22.

## Revendications

1. Composant fémoral d'une prothèse du genou, comportant une partie de trochlée (3) et deux condyles (2), un trajet trochléen étant défini dans la partie de trochlée dans la surface extérieure du composant, et un ensemble de faces planes (5, 6, 7, 8, 9) intérieures destinées à venir en contact avec des faces réséquées correspondantes de l'extrémité d'un fémur définissant un cage ouverte intérieure dans la surface intérieure du composant, lesdites faces planes intérieures définissant entre elles des arêtes (15, 16, 17, 18) intérieures, les condyles définissant une ligne de référence (22) lorsqu'ils sont posés sur un plan horizontal, correspondant à la ligne de contact avec ce plan, les arêtes en projection orthogonale sur le plan médio-latéral faisant un angle d'inclinaison (α) non nul avec la ligne (22) de référence dans le plan médio-latéral, **caractérisé en ce que** la projection (4) perpendiculaire du trajet trochléen dans le plan médio-latéral est perpendiculaire à la ligne (22) de référence.

2. Composant suivant la revendication 1, **caractérisé en ce que** la cage ouverte intérieure comporte cinq faces planes.

3. Composant suivant la revendication 1 ou 2 **caractérisé en ce que** les condyles sont extérieurement de forme sphérique.

4. Composant suivant l'une des revendication 1 à 3, **caractérisé en ce que** l'angle d'inclinaison correspond à l'angle dont doit être tourné le fémur dans le cas de la méthode dite de la rotation externe du fémur, de sorte que cette méthode n'est plus nécessaire.

5. Composant suivant l'une des revendications 1 à 4, **caractérisé en ce que** l'angle d'inclinaison est compris en valeur absolue entre 1° et 10°, de préférence entre 2° et 5° ; notamment est égal à 3°.

6. Couple de composants fémoraux suivant l'une des revendications précédentes, **caractérisé en ce que** les angles d'inclinaison des deux composants sont de signes trigonométriques opposés.

7. Couple suivant la revendication 6, **caractérisé en ce que** les angles d'inclinaison des deux composants ont la même valeur absolue.

8. Prothèse du genou comportant un plateau tibial et un composant fémoral suivant l'une des revendications 1 à 5.

## Patentansprüche

1. Oberschenkelknochen-Bauteil einer Knieprothese mit einem Trochleabereich (3) und zwei Kondylen (2), wobei eine Trochlea-Bahn im Trochleabereich in der Außenflache des Bauteils definiert ist, und mit einer Einheit von ebenen inneren Flachen (5, 6, 7, 8, 9), die dazu bestimmt sind, mit entsprechenden resezierten Flächen des Endes eines Oberschenkelknochens in Kontakt zu kommen, die einen offenen inneren Käfig in der Innenfläche des Bauteils bilden, wobei die inneren ebenen Flachen miteinander innere Kanten (15, 16, 17, 18) bilden, wobei die Kondyle eine Bezugslinie (22) definieren, wenn sie auf eine waagrechte Ebene aufgebracht werden, die der Kontaktlinie mit dieser Ebene entspricht, wobei die Kanten in der orthogonalen Projektion auf die medial-laterale Ebene einen Neigungswinkel (α) ungleich Null mit der Bezugslinie (22) in der medial-lateralen Ebene bilden, **dadurch gekennzeichnet, dass** die senkrechte Projektion (4) der Trochlea-Bahn in der medial-lateralen Ebene senkrecht zur Bezugslinie (22) ist.

2. Bauteil nach Anspruch 1, **dadurch gekennzeichnet, dass** der offene innere Käfig fünf ebene Flachen aufweist.

3. Bauteil nach Anspruch 1 oder 2, **dadurch gekennzeichnet, dass** die Kondyle außen kugelförmig sind.

4. Bauteil nach einem der Ansprüche 1 bis 3, **dadurch gekennzeichnet, dass** der Neigungswinkel dem Winkel entspricht, um den der Oberschenkelknochen im Fall der Methode mit externer Drehung des Oberschenkelknochens gedreht werden muss, so dass diese Methode nicht mehr notwendig ist.

5. Bauteil nach einem der Anspruche 1 bis 4, **dadurch gekennzeichnet, dass** der Neigungswinkel im Absolutwert zwischen 1° und 10°, vorzugsweise zwischen 2° und 5° liegt und insbesondere gleich 3° ist.

6. Paar von Oberschenkelknochen-Bauteilen nach einem der vorhergehenden Anspruche, **dadurch gekennzeichnet, dass** die Neigungswinkel der beiden Bauteile entgegengesetzte trigonometrische Vorzeichen haben

7. Paar nach Anspruch 6, **dadurch gekennzeichnet, dass** die Neigungswinkel der beiden Bauteile den gleichen Absolutwert haben

8. Knieprothese, die ein Tibiaplateau und ein Oberschenkelknochen-Bauteil nach einem der Anspruche 1 bis 5 aufweist.

## Claims

1. A femoral component of a knee prosthetic including a trochlean part (3) and two condyles (2), wherein
a trochlean trajectory is defined within the trochlean portion in the external surface of the component;
a set of internal flat sides (5,6,7,8,9) are implemented to make contact with corresponding re-cut sides of the extremity of the femur and define an internal open cage within the internal surface of said component and further define edges (15,16,17,18) between themselves;
wherein said condyle, when contacting a horizontal plane, defines a reference line (22) corresponding to the contact line with said plane,
the edges have a tilt angle (α) with a value different from zero relative to said reference line (22) when orthogonally projected on the medial lateral plane, **characterised in that** the perpendicular projection (4) of said trochlean trajectory in the medio lateral plane is perpendicular to said reference line (22).

2. The component according to claim 1, **characterised in that** said internal open cage includes five flat sides.

3. The component according to claim 1 or 2, **characterised in that** the external shape of said condyle is spherical.

4. Component according to one of claims 1 to 3, **characterised in that** said tilt angle corresponds to the angle of which said femur must be rotated in the case of said femur external rotation method, so that said method is no longer necessary.

5. The component according to one of claims 1 to 3, **characterised in that** said tilt angle has an absolute value between one and ten degrees, preferably between 2° and 5°, in particular 3°.

6. A couple of femoral components according to one of the previous claims, **characterised in that** the respective tilt angles of both components have opposite trigonometric sides.

7. A couple of femoral components according to claim 6, **characterised in that** said respective tilt angles of both components have the same absolute value.

8. A knee prosthetic including a tibia plate and a femoral component according to one of claims 1 to 5.
